# EUROPEAN PATENT APPLICATION

(11) **EP 2 832 343 A1**
(43) Date of publication of application: **04.02.2015**
(21) Application number: 13767626.8
(22) Date of filing: 27.03.2013
(51) Int. Cl.: A61K 8/25, A61K 8/02, A61K 8/92, A61Q 5/00, A61Q 5/06

(54) **POWDERY SOLID HAIR COMPOSITION, POWDERY SOLID HAIR COSMETIC, METHOD FOR PRODUCING POWDERY SOLID HAIR COSMETIC, AND METHOD FOR HAIRDRESSING USING POWDERY SOLID HAIR COSMETIC**

(30) Priority: 28.03.2012 JP 2012073890
(71) Applicant: Mandom Corporation, Osaka-shi, Osaka 540-8530 (JP)
(72) Inventor: ISHII, Takeharu, Osaka-shi Osaka 540-8530 (JP); ASADA, Takuji, Osaka-shi Osaka 540-8530 (JP)
(74) Representative: TBK
(86) International application number: PCT/JP2013/059062
(87) International publication number: WO 2013/146908

(57) **Abstract**

The present invention provides a powdery solid hair cosmetic that enables easy hairdressing, enables hairdressing into an intended hairstyle without sticky feeling, can impart a natural texture, and has an unprecedented, novel form. The powdery solid composition for hair of the present invention is in a powdery solid form, is used for hair, and contains a powder and an oil.

## Description

### Technical Field

The present invention relates to a powdery solid composition for hair, a powdery solid hair cosmetic including the composition and a container, a method for producing the cosmetic, and a method for hairdressing using the cosmetic.

### Background Art

Cosmetics for hairdressing are exemplified by liquid, gel, foamy, and mist hair cosmetics containing a film forming resin as a main functional component for hairdressing (for example, see Patent Documents 1 to 4). The cosmetics are also exemplified by cream and wax hair cosmetics containing an oil such as waxes and hydrocarbon oils as a main functional component for hairdressing (for example, see Patent Documents 5 and 6). At the present time, typical hair cosmetics contain a film forming resin or an oil.

The existing hair cosmetics containing a film forming resin or an oil can sufficiently achieve excellent hairdressing function but have a problem of causing sticky feeling and a problem of being difficult to impart a natural texture. In order to solve the problems, various attempts have been made.

There are various methods for hairdressing using hair cosmetics depending on forms of the cosmetics. Typically, a method of hairdressing by using a comb and a method of hairdressing with the palms and fingers of hands have been attempted, for example.

However, the method of hairdressing by using a comb can achieve easy hairdressing of the whole hair but fails to sufficiently achieve hairdressing in fine detail. The method of hairdressing with the palms and fingers in place of a comb can sufficiently achieve hairdressing in fine detail but fails to achieve easy hairdressing.

As described above, conventional hair cosmetics are difficult to easily, sufficiently achieve hairdressing in fine detail. In particular, for example, away from home, it is more difficult to easily, sufficiently achieve hairdressing in fine detail, and easy re-dressing is impossible. In consideration of such hair cosmetics and the circumstances, there is a demand for a hair cosmetic having an unprecedented, novel form and enabling easy hairdressing.

### Citation List

### Patent Literature

Patent Document 1: JP-A No. 9-301838
Patent Document 2: JP-A No. 2001-335425
Patent Document 3: JP-A No. 2000-204025
Patent Document 4: JP-A No. 2011-251918
Patent Document 5: JP-A No. 2009-173602
Patent Document 6: JP-A No. 2002-114652

### Summary of Invention

### Technical Problem

The inventors of the present invention have carried out intensive studies in order to develop a hair cosmetic having an unprecedented, novel form and have found that direct application of a powder to hair surprisingly enables the hairdressing into an intended hairstyle without sticky feeling and can impart a natural texture (natural finish).

In addition, the inventors of the present invention have found that a powdery solid hair cosmetic having an unprecedented, novel form can solve the problems of conventional hair cosmetics, including (1) hairdressing by using a comb achieving hairdressing of the whole hair but failing to sufficiently achieve hairdressing in fine detail, (2) hairdressing with the palms and fingers failing to achieve easy hairdressing, and (3) re-dressing failing to be easily performed, for example, away from home, and have completed the present invention.

An object of the present invention is to provide a powdery solid composition for hair that enables easy hairdressing, enables hairdressing into an intended hairstyle without sticky feeling, can impart a natural texture, and has an unprecedented, novel form. The present invention also provides a powdery solid hair cosmetic including the powdery solid hair cosmetic and a container, a method for producing the powdery solid hair cosmetic, and a method for hairdressing using the powdery solid hair cosmetic.

### Solution to Problem

In order to solve the problem, the present invention provides a powdery solid composition for hair in a powdery solid form used for hair, and the powdery solid composition includes a powder and an oil.

In the powdery solid composition for hair of the present invention, it is preferable that the powder be contained in an amount of 30 to 95% by mass.

In the powdery solid composition for hair of the present invention, it is preferable that the oil be contained in an amount of 5 to 70% by mass.

A powdery solid hair cosmetic of the present invention includes the powdery solid composition for hair and a container storing the powdery solid composition for hair.

The method for producing a powdery solid hair cosmetic of the present invention is a method for producing the powdery solid hair cosmetic above. The method includes a first step of mixing and stirring the powder and the oil to yield a mixture as the powdery solid composition for hair, a second step of directly charging the mixture obtained in the first step into the container or charging the mixture into an inner plate to be installed in the container, and a third step of compression molding the charged mixture or molding the charged mixture, the mixture not being subjected to compression molding, by a molding method except the compression molding.

A method for hairdressing using the powdery solid hair cosmetic of the present invention includes a first step of rubbing a surface of the powdery solid composition for hair with a fingertip or an applicator to take the powdery solid composition for hair, a second step of applying the taken powdery solid composition for hair onto hair with the fingertip or the applicator, and a third step of rubbing the powdery solid composition for hair applied onto the hair against the hair with the fingertip or the palm of a hand to perform hairdressing.

### Advantageous Effects of Invention

The powdery solid composition for hair of the present invention is in a powdery solid form. From the surface of the composition, a minimum mount of the composition needed for hairdressing can be taken. The composition can be applied thinly on hair. Therefore, the powdery solid composition for hair has the effect of enabling hairdressing into an intended hairstyle without sticky feeling and imparting a natural texture.

The powdery solid hair cosmetic of the present invention including the composition stored in a container has the effect of enabling easy hairdressing without using any comb during the hairdressing. The powdery solid hair cosmetic is excellent in convenience and thus enables easy re-dressing even, for example, away from home.

The method for producing the powdery solid hair cosmetic of the present invention can provide a powdery solid hair cosmetic in an unprecedented, novel form. The method for hairdressing using the powdery solid hair cosmetic of the present invention has the effect of enabling easy hairdressing from the whole hair to a detailed region.

### Description of Embodiments

The powdery solid composition for hair of the present invention is in a powdery solid form and is used for hair. The powdery solid composition for hair of the present invention is applied to hair. The powdery solid composition for hair of the present invention includes a powder and an oil.

Examples of the powder include inorganic powders and organic powders. Examples of the shape of the powder include a spherical shape, a plate-like shape, an acicular shape, and an indefinite shape. The powder may have any shape, which is not particularly limited.

Specific examples of the inorganic powder include silica, calcium silicate, magnesium silicate, aluminum silicate, synthetic aluminum silicate, natural aluminum silicate, magnesium aluminum silicate, talc, kaolin, mica, mica titanium, sericite, bentonite, calcium carbonate, magnesium carbonate, calcium sulfate, barium sulfate, magnesium sulfate, zinc oxide, titanium oxide, aluminum oxide, zirconium oxide, magnesium oxide, iron oxide, yellow iron oxide, black iron oxide, an iron oxide-titanium oxide sintered product, a red iron oxide-coated mica, a red iron oxide-coated mica titanium, a titanium oxide-coated silica, chromium oxide, tin oxide, alumina, bismuth oxychloride, ultramarine, iron blue, calamine, borosilicic acid, carbon black, cobalt titanate, soft calcium, soft magnesium, heavy calcium, heavy magnesium, aluminum powder, boron nitride, hydroxyapatite, borosilicate (Ca/Na), and zeolite.

In the present invention, the powder may be silica-coated powders and the like prepared by applying silica onto the surface of the powders above including talc, kaolin, mica, titanium oxide, aluminum oxide, zinc oxide, and cerium oxide.

Specific examples of the organic powder include powders of nylons such as nylon 6, nylon 12, and nylon 66; powders of polyolefins such as polyethylene and polypropylene; powders of polyacrylates such as polymethyl methacrylate; powders of silicone resins such as polymethylsilsesquioxane and dimethyl polysiloxane polymers; powders of fluorine resins such as polytetrafluoroethylene; polystyrene powders, polyurethane powders, polyepoxy powders, polyethylene terephthalate, polypropylene terephthalate, polycarbonate, celluloid, cellulose, crystalline cellulose, acetyl cellulose, chitin, chitosan, polysaccharides, protein powders, lauroyl lysine, vinyl resins, urea resins, (PET/polyolefin) laminates, and (PET/Al/epoxy resin) laminates.

The powders may be used singly or in an appropriate combination of two or more of them. The amount of the powder contained in the powdery solid composition for hair of the present invention is not particularly limited and is appropriately adjusted so as to sufficiently impart an intended effect. In order to produce a good powdery solid, the powder is typically, preferably contained in an amount of 20% by mass or more, more preferably 30% by mass or more, preferably 99% by mass or less, and more preferably 95% by mass or less relative to 100% by mass of the composition . In order to produce a good powdery solid, the powder is preferably contained in an amount of 20 to 99% by mass and more preferably 30 to 95% by mass relative to 100% by mass of the composition.

In the present invention, among the powders, at least the inorganic powder is preferably used, and among the inorganic powders, at least silica is more preferably used in order to perform hairdressing with much less sticky feeling.

The inorganic powder is preferably contained in an amount of 1% by mass or more, more preferably 5% by mass or more, even more preferably 10% by mass or more, particularly preferably 20% by mass or more, most preferably 30% by mass or more, preferably 99% by mass or less, and more preferably 95% by mass or less relative to 100% by mass of the composition. The inorganic powder may be contained in an amount of 60% by mass or less, 40% by mass or less, or 30% by mass or less relative to 100% by mass of the composition.

The silica is not particularly limited, and a silica capable of sufficiently imparting an intended effect is appropriately selected. Specifically, the silica may be a hydrophilic silica having silanol groups and siloxane on the surface without treatment and may be a hydrophobic silica obtained by hydrophobic treatment of the surface of a hydrophilic silica.

Any treatment agents may be used for the hydrophobic treatment, and specific examples of the treatment agent include organic silyl compounds such as methyltrichlorosilane, dimethyldichlorosilane, trimethylchlorosilane, hexamethyldisilazane, methyltrialkoxysilane, dimethyldialkoxysilane, trimethylalkoxysilane, ethyltrichlorosilane, propyltrichlorosilane, hexyltrichlorosilane, long-chain alkyltrichlorosilane, ethyltrialkoxysilane, propyltrialkoxysilane, hexyltrialkoxysilane, long-chain alkyltrialkoxysilane, methacrylsilane, fluoroalkylsilane, and perfluoroalkylsilane; and silicone compounds such as dimethylpolysiloxane, methylphenylpolysiloxane, methylhydrogenpolysiloxane, and amino-modified silicone. Any known treatment methods capable of performing hydrophobic treatment may be employed.

A commercially available silica may be used without treatment. Examples of the commercially available hydrophilic silica include AEROSIL 50, AEROSIL 90G, AEROSIL 130, AEROSIL 150, AEROSIL 200, AEROSIL 300, AEROSIL 380, AEROSIL 200V, and AEROSIL OX50 (trade name, each manufactured by NIPPON AEROSIL CO., LTD.); SUNSPHERE H-31, SUNSPHERE H-51, SUNSPHERE H-121, SUNSPHERE H-201, SUNSPHERE H-32, SUNSPHERE H-52, SUNSPHERE H-122, SUNSPHERE H-33, and SUNSPHERE H-53 (trade name, each manufactured by AGC Si-Tech. Co., Ltd.); and Sylysia 320, Sylysia 310P, Sylysia 250, Sylysia 250N, Sylysia 350, Sylysia 370, Sylysia 380, Sylysia 420, Sylysia 430, Sylysia 440, Sylysia 450, and Sylysia 470 (trade name, each manufactured by Fuji Silysia Chemical Ltd.).

Examples of the commercially available hydrophobic silica include AEROSIL RX200, AEROSIL R8200, AEROSIL RX300, AEROSIL R812, AEROSIL R812S, AEROSIL RY200, and AEROSIL RY200S (trade name, each manufactured by NIPPON AEROSIL CO., LTD.); VM-2270 Aerogel Fine particles (trade name, manufactured by Dow Corning Toray Co., Ltd.); and SUNSPHERE H-51-ET, SUNSPHERE H-52-ET, and SUNSPHERE H-121-ET (trade name, each manufactured by AGC Si-Tech. Co., Ltd.).

The amount of the silica is not particularly limited and is appropriately adjusted so as to sufficiently impart an intended effect. In order to perform hairdressing without sticky feeling and to enable re-dressing, the silica is typically, preferably contained in an amount of 1% by mass or more, more preferably 5% by mass or more, even more preferably 10% by mass or more, preferably 60% by mass or less, more preferably 40% by mass or less, and even more preferably 30% by mass or less relative to 100% by mass of the composition. The silica is preferably contained in an amount of 1 to 60% by mass, more preferably 5 to 40% by mass, and even more preferably 10 to 30% by mass relative to 100% by mass of the composition. The silica may be contained in an amount of less than 1% by mass relative to 100% by mass of the composition, but if containing the silica in an amount of less than 1% by mass, the composition is likely to reduce the hairdressing performance.

Examples of the oil include oils and fats, waxes, hydrocarbon oils, higher fatty acids, higher alcohols, fatty acid ester oils, and silicone oils.

Specific examples of the oil and fat include liquid oils and fats such as sunflower oil, avocado oil, cottonseed oil, soybean oil, olive oil, macadamia nut oil, peanut oil, corn oil, rapeseed oil, sesame oil, safflower oil, rice bran oil, castor oil, jojoba oil, camellia oil, and mink oil; and solid oils and fats such as coconut oil, palm oil, palm kernel oil, cacao butter, hardened oil, and hydrogenated castor oil.

Specific examples of the waxes include carnauba wax, candelilla wax, beeswax, rice bran wax, montan wax, shellac wax, spermaceti wax, jojoba wax, lanolin, liquid lanolin, reduced lanolin, and hard lanolin.

Specific examples of the hydrocarbon oil include ceresin, paraffin, liquid paraffin, liquid isoparaffin, solid paraffin, microcrystalline wax, Fischer-Tropsch wax, polyethylene powder, polyethylene wax, vaseline, and squalene.

Specific examples of the higher fatty acid include lauric acid, myristic acid, palmitic acid, stearic acid, oleic acid, behenic acid, undecylenic acid, isostearic acid, linoleic acid, linolenic acid, and 12-hydroxystearic acid.

Specific examples of the higher alcohol include lauryl alcohol, myristyl alcohol, cetyl alcohol, stearyl alcohol, oleyl alcohol, lanolin alcohol, behenyl alcohol, cetostearyl alcohol, and isostearyl alcohol.

Specific examples of the fatty acid ester oil include ethyl oleate, isopropyl myristate, isopropyl palmitate, butyl stearate, myristyl myristate, cetyl myristate, cetyl palmitate, stearyl stearate, decyl oleate, oleyl oleate, octyldodecyl myristate, octyldodecyl oleate, ethyl isostearate, isocetyl stearate, isopropyl isostearate, isocetyl isostearate, cetyl 2-ethylhexanoate, cetostearyl 2-ethylhexanoate, neopentyl glycol dicaproate, neopentyl glycol dioctanoate, glyceryl tri-2-ethylhexanoate, glyceryl trioctanoate, glyceryl triisopalmitate, glyceryl triisostearate, trimethylolpropane tri-2-ethylhexanoate, trimethylolpropane triisostearate, pentaerythritol tetra-2-ethylhexanoate, cetyl octanoate, isocetyl octanoate, isostearyl octanoate, isostearyl sebacate, octyldodecyl isostearate, octyldodecyl dimethyloctanoate, myristyl lactate, cetyl lactate, and diisostearyl malate.

Specific examples of the silicone oil include methylpolysiloxane, highly polymerized methylpolysiloxane, octamethyltrisiloxane, decamethyltetrasiloxane, methylphenylpolysiloxane, octamethylcyclotetrasiloxane, decamethylcyclopentasiloxane, dodecamethylcyclohexasiloxane, methylcyclopolysiloxane, alcohol-modified silicone, alkyl-modified silicone, amino-modified silicone, polyether-modified silicone, and fluorine-modified silicone.

The oils may be used singly or in an appropriate combination of two or more of them. The powdery solid composition for hair of the present invention contains the oil in any amount, and the amount is appropriately adjusted so as to sufficiently impart an intended effect. In order to produce a good powdery solid, the oil is typically, preferably contained in an amount of 1% by mass or more, more preferably 5% by mass or more, preferably 80% by mass or less, and more preferably 70% by mass or less relative to 100% by mass of the composition. In order to produce a good powdery solid, the oil is preferably contained in an amount of 1 to 80% by mass and more preferably 5 to 70% by mass relative to 100% by mass of the composition. The oil may be contained in an amount of 2% by mass or more relative to 100% by mass of the composition.

The powdery solid composition for hair of the present invention may contain components typically used in cosmetics in addition to the components above as long as the advantageous effects of the present invention are not impaired. Examples of the component include nonionic surfactants, anion surfactants, cationic surfactants, amphoteric surfactants, polyhydric alcohols, water-soluble polymers, moisturizers, antioxidants, ultraviolet absorbers, chelating agents, antiseptics, vitamins, animal and plant extracts, and perfumes. These components may be appropriately added depending on a purpose.

The powdery solid composition for hair of the present invention has the effect of enabling hairdressing without sticky feeling and imparting a natural texture to hair, and thus is suitably used for hairdressing.

A powdery solid hair cosmetic of the present invention includes the powdery solid composition for hair and a container storing the powdery solid composition for hair. By providing the powdery solid composition for hair of the present invention in a powdery solid hair cosmetic state, hairdressing can be easily performed without using any comb during the hairdressing.

The container used for the powdery solid hair cosmetic is not particularly limited, and a container capable of storing a powdery solid composition is appropriately selected. Examples of the container include jar containers and compact containers with a removable lid, jar containers and compact containers with a hinged lid, and stick containers.

In the present invention, a compact container with a removable lid or a compact container with a hinged lid is preferably used because such a container is excellent in convenience and enables easy re-dressing even away from home, for example.

The powdery solid hair cosmetic of the present invention may be either in a state in which the powdery solid composition for hair is directly charged into the container or in a state in which the powdery solid composition for hair is charged into an inner plate installed in the container. The inner plate is preferably, detachably installed in the container. The powdery solid composition for hair may be molded into any shapes, which are designed to fit the shape of a container or an inner plate to be used.

The material of the container used for the cosmetic is not particularly limited and is exemplified by PET resins, AS resins, ABS resins, and polycarbonate resins. The material of the inner plate is also not particularly limited and may be made of either a metal or a resin.

The powdery solid hair cosmetic of the present invention can be produced by a known method. Specific examples of the production method include a production method of compression molding the powdery solid composition (mixture) for hair and a production method of molding the powdery solid composition (mixture) for hair that is not subjected to compression molding by a molding method except the compression molding.

The production method by compression molding may be either a dry method or a wet method. The production method by compression molding is specifically exemplified by a production method that includes a first step of mixing and stirring the powder and the oil to yield a mixture as the powdery solid composition for hair, a second step of directly charging the mixture obtained in the first step into the container or charging the mixture into an inner plate to be installed in the container, and a third step of compression molding the charged mixture.

The mixer used in the first step is not particularly limited, and a mixer capable of homogeneously mixing the powder with the oil is appropriately selected. Examples of the mixer include Henschel mixers, blender mixers, V-mixers, ribbon mixers, Nauter mixers, and high speed mixers.

In the present invention, the mixture obtained in the first step may be charged into the container or the inner plate without treatment. In order to increase the uniformity during charging, the mixture may be pulverized with a pulverizer such as a hammer mill, a pin mill, and an atomizer, then the pulverized product may be passed through a sieve, and the sieved mixture may be charged. In the second step, the mixture may be charged while being vibrated in order to further increase the uniformity during charging.

In the third step, the pressure applied during compression molding is not particularly limited and is appropriately adjusted so as to give a powdery solid having an intended hardness. The pressure applied during compression molding is preferably 0.01 MPa or more, more preferably 0.05 MPa or more, preferably 4 MPa or less, and more preferably 3 MPa or less.

For the compression molding by a dry method, the production method above can be employed. For the compression molding by a wet method, the mixture obtained in the first step is preferably mixed with a volatile solvent into a slurry. Alternatively, the mixture may pulverized with a pulverizer such as a hammer mill, a pin mill, and an atomizer, then the pulverized product may be passed through a sieve, and the sieved mixture may be processed into a slurry.

The volatile solvent used for preparing a slurry may be any solvent capable of making the mixture of the powder and the oil into a slurry. Examples of the volatile solvent include water, ethyl alcohol, isopropyl alcohol, n-butanol, isoparaffin, light isoparaffin, octamethylcyclotetrasiloxane, decamethylcyclopentasiloxane, dodecamethylcyclohexasiloxane, octamethyltrisiloxane, tetradecamethylhexasiloxane, and low-viscosity methylpolysiloxane. The volatile solvents may be used singly or in an appropriate combination of two or more of them.

In order to make the mixture into a slurry with the volatile solvent, the volatile solvent is preferably used in an amount of 10 to 150 parts by mass relative to 100 parts by mass of the mixture, and the slurry prepared in the mass ratio is more readily molded into various shapes.

For the compression molding by a wet method, in order to remove the volatile solvent, vacuum aspiration drying is preferably performed during the compression molding, and the molded product is preferably further dried by, for example, air drying, drying by heating, or warm air drying.

The production method without compression molding is exemplified by a production method that includes a first step of mixing and stirring the powder and the oil to yield a mixture as the powdery solid composition for hair, a second step of directly charging the mixture obtained in the first step into the container or charging the mixture into an inner plate to be installed in the container, and a third step of molding the charged mixture that is not subjected to compression molding by a molding method except the compression molding.

Specific examples of the third step without compression molding include a step of molding the mixture by drying, for example, air drying, dried by heating, or warm air drying and a step of molding the mixture by solidification. The solidification of the mixture may be performed by either a method that includes charging the mixture, then warming and dissolving the mixture, and solidifying the mixture or a method that includes warming and dissolving the mixture, then charging the mixture, and solidifying the mixture.

A method for hairdressing using the powdery solid hair cosmetic of the present invention includes a first step of rubbing the surface of the powdery solid composition for hair with a fingertip or an applicator to take the powdery solid composition for hair, a second step of applying the taken powdery solid composition for hair onto hair with the fingertip or the applicator, and a third step of rubbing the powdery solid composition for hair applied onto the hair against the hair with the fingertip or the palm of a hand to perform hairdressing.

The most important feature of the method for hairdressing of the present invention is rubbing the surface of the powdery solid composition for hair to take the composition and performing hairdressing. The means for taking the composition by rubbing is not particularly limited, but the composition is preferably taken by rubbing with fingertips or an applicator. By using the fingertips, the composition can be easily applied to a detailed region, and this enables sufficient hairdressing in fine detail. By using the applicator, the composition can be easily applied to the whole hair, and this enables hairdressing of the whole hair.

The applicator to be used may be any applicator with which the powdery solid composition for hair can be taken by rubbing the surface of the composition. Examples of the applicator include bristle brushes and puffs for powdery solid.

According to the method for hairdressing of the present invention, by rubbing the composition applied to hair against the hair with the fingertips or palms, the hairdressing can be performed into an intended hairstyle. The method for hairdressing above enables easy hairdressing from the whole hair to a detailed region.

The powdery solid composition for hair and the powdery solid hair cosmetic including the composition and a container of the present invention provide an unprecedented, novel form that enables hairdressing into an intended hairstyle without sticky feeling and can impart a natural texture. Therefore, conventional hair cosmetics can be replaced by the composition and the cosmetic.

### Examples

The present invention will next be described in further detail with reference to examples, but the invention is not limited to these examples alone. The mixing amounts are in terms of "% by mass" unless otherwise specified.

### (Preparation of Sample)

In accordance with the formulation shown in Table 1, each powdery solid composition for hair of Examples 1 to 5 was prepared by the production process and was subjected to the evaluations below. The results are also shown in Table 1. Each evaluation was carried out in a constant temperature and humidity condition at a temperature of 23°C and a humidity of 60%.

### (Production Process)

The powders described in Table 1 were mixed with a Henschel mixer, and then the oils were added. The whole was further mixed and stirred to yield a mixture. Next, the mixture was charged into an inner plate to be installed in a compact container and was compression molded.

### (Test Example 1: Evaluation of Sticky Feeling and Hairdressing Performance)

Twenty panelists for sensory evaluation performed hairdressing of a wig (Lesson mannequin: manufactured by Yukari Japan Co., Ltd.) by using each sample obtained in Examples by the hairdressing method below and performed sensory evaluation of "sticky feeling" and "hairdressing performance" during the hairdressing in accordance with the evaluation standards below.

### (Method for Hairdressing)

The powdery solid composition for hair was taken by rubbing the surface of the composition with the fingertips, and the taken composition was applied to hair with the fingertips. Next, the composition applied to the hair was rubbed against the hair with the fingertips or palms to perform hairdressing.

### <Evaluation Standard for Sticky Feeling>

A (excellent): Of 20 panelists, 16 or more reported that the hairdressing was performed without sticky feeling.
B (good): Of 20 panelists, 10 to 15 reported that the hairdressing was performed without sticky feeling.
C (poor): Of 20 panelists, 9 or less reported that the hairdressing was performed without sticky feeling.

### <Evaluation Standard for Hairdressing Performance>

A (excellent): Of 20 panelists, 16 or more reported that the hairdressing was performed into an intended hairstyle.
B (good): Of 20 panelists, 10 to 15 reported that the hairdressing was performed into an intended hairstyle.
C (poor): Of 20 panelists, 9 or less reported that the hairdressing was performed into an intended hairstyle.

### (Test Example 2: Evaluation of Texture)

Twenty panelists for sensory evaluation who had evaluated Test Example 1 visually evaluated "hair texture" at 10 minutes after the evaluation of Test Example 1 in accordance with the evaluation standard below.

### <Evaluation Standard for Hair Texture>

A (excellent): Of 20 panelists, 16 or more reported a natural texture without floating powder.
B (good): Of 20 panelists, 10 to 15 reported a natural texture without floating powder.
C (poor): Of 20 panelists, 9 or less reported a natural texture without floating powder.

**[Table 1]**

| | Example | | | | |
|---|---|---|---|---|---|
| | 1 | 2 | 3 | 4 | 5 |
| Silica | 5.0 | 10.0 | 20.0 | 30.0 | 40.0 |
| Talc | 67.0 | 30.0 | 42.0 | 52.0 | 42.0 |
| Mica | 20.0 | - | 30.0 | - | - |
| Kaolin | - | 52.0 | - | 10.0 | 10.0 |
| Squalene | 6.0 | 6.0 | 6.0 | - | - |
| Liquid paraffin | - | - | - | 6.0 | 6.0 |
| Octyldodecyl myristate | 2.0 | 2.0 | 2.0 | 2.0 | 2.0 |
| Total | 100.0 | 100.0 | 100.0 | 100.0 | 100.0 |
| Sticky feeling | A | A | A | A | A |
| Hairdressing performance | A | A | A | A | A |
| Hair texture | A | A | A | A | A |

The results shown in Table 1 indicate that the powdery solid composition for hair described in each Example enabled hairdressing into an intended hair style without sticky feeling, and a natural texture was observed without floating powder after the hairdressing. In Examples, specifically, the mixture was charged into an inner plate to be installed in a container. A case by directly charging the mixture into a container was also ascertained to give excellent results. In Examples, specifically, the charged mixture was compression molded. A case by molding the charged mixture that was not subjected to compression molding by a molding method except the compression molding was also ascertained to give excellent results. In Examples, specifically, the composition was taken by rubbing with the fingertips and was applied to hair with the fingertips. A composition taken with an applicator in place of the fingertips was also ascertained to give excellent results.

Mixing examples for the powdery solid composition for hair and the powdery solid hair cosmetic including the composition and a container pertaining to the present invention will be shown below. The amounts are in terms of % by mass.

**[Table 2]**

| (Mixing Example 1) | |
|---|---|
| Silica | 15.0 |
| Talc | 35.0 |
| Mica | 20.0 |
| Kaolin | 22.0 |
| Liquid paraffin | 6.0 |
| Octyldodecyl myristate | 2.0 |
| Total | 100.0 |

In Mixing Example 1, the powders were mixed with a Henschel mixer, and then the oils were added. The whole was further mixed and stirred to yield a mixture. Next, the mixture was charged into an inner plate to be installed in a compact container and was compression molded.

**[Table 3]**

| (Mixing Example 2) | |
|---|---|
| Silica | 10.0 |
| Talc | 10.0 |
| Solid paraffin | 3.0 |
| Microcrystalline wax | 6.0 |
| Beeswax | 2.0 |
| Vaseline | 12.0 |
| Liquid paraffin | 20.0 |
| Isopropyl palmitate | 37.0 |
| Total | 100.0 |

In Mixing Example 2, the powders were mixed with a Henschel mixer, and then the mixture was added to the oils that had been warmed and dissolved. The whole was further mixed and stirred to yield a mixture. Next, the mixture was charged into an inner plate to be installed in a compact container, then was allowed to cool to room temperature, and was solidified.

**[Table 4]**

| (Mixing Example 3) | |
|---|---|
| Silica | 20.0 |
| Talc | 10.0 |
| Solid paraffin | 3.0 |
| Microcrystalline wax | 6.0 |
| Beeswax | 2.0 |
| Vaseline | 12.0 |
| Liquid paraffin | 15.0 |
| Isopropyl palmitate | 32.0 |
| Total | 100.0 |

In Mixing Example 3, the powders were mixed with a Henschel mixer, and then the mixture was added to the oils that had been warmed and dissolved. The whole was further mixed and stirred to yield a mixture. Next, the mixture was charged into an inner plate to be installed in a compact container and was compression molded.

## Claims

1. A powdery solid composition for hair in a powdery solid form used for hair, the powdery solid composition comprising:
a powder; and
an oil.

2. The powdery solid composition for hair according to claim 1, wherein the powder is contained in an amount of 30 to 95% by mass.

3. The powdery solid composition for hair according to claim 1 or 2, wherein the oil is contained in an amount of 5 to 70% by mass.

4. A powdery solid hair cosmetic comprising:
the powdery solid composition for hair according to any one of claims 1 to 3; and
a container storing the powdery solid composition for hair.

5. A method for producing the powdery solid hair cosmetic according to claim 4, the method comprising:
a first step of mixing and stirring the powder and the oil to yield a mixture as the powdery solid composition for hair;
a second step of directly charging the mixture obtained in the first step into the container or charging the mixture into an inner plate to be installed in the container; and
a third step of compression molding the charged mixture or molding the charged mixture, the mixture not being subjected to compression molding, by a molding method except the compression molding.

6. A method for hairdressing using the powdery solid hair cosmetic according to claim 4, the method comprising:
a first step of rubbing a surface of the powdery solid composition for hair with a fingertip or an applicator to take the powdery solid composition for hair;
a second step of applying the taken powdery solid composition for hair onto hair with the fingertip or the applicator; and
a third step of rubbing the powdery solid composition for hair applied onto the hair against the hair with the fingertip or the palm of a hand to perform hairdressing.
